(19) Europäisches Patentamt
European Patent Office
Office européen des brevets

(11) Numéro de publication : **0 452 182 B1**

# (12) FASCICULE DE BREVET EUROPEEN

(45) Date de publication du fascicule du brevet : **03.08.94 Bulletin 94/31**

(51) Int. Cl.$^5$ : **C07C 1/20,** B01J 23/02, B01J 23/08, B01J 23/10, B01J 23/12, B01J 21/14

(21) Numéro de dépôt : **91400826.3**

(22) Date de dépôt : **27.03.91**

## (54) Procédé d'obtention d'au moins une oléfine tertiaire par décomposition de l'éther correspondant.

(30) Priorité : **09.04.90 FR 9004498**

(43) Date de publication de la demande : **16.10.91 Bulletin 91/42**

(45) Mention de la délivrance du brevet : **03.08.94 Bulletin 94/31**

(84) Etats contractants désignés : **AT BE CH DE DK ES FR GB GR IT LI NL SE**

(56) Documents cités :
**EP-A- 0 050 992**
**WO-A-87/00166**
**US-A- 4 254 296**

(73) Titulaire : **INSTITUT FRANCAIS DU PETROLE**
**4, Avenue de Bois Préau**
**F-92506 Rueil-Malmaison Cédex (FR)**

(72) Inventeur : **Chaumette, Patrick**
**34, Côte de la Jonchère**
**F-78380 Bougival (FR)**
Inventeur : **Martino, Germain**
**Bât. Condé,**
**80, av. F. Lefebvre**
**F-78300 Poissy (FR)**
Inventeur : **Verdon, Catherine**
**54, rue Eugène Labiche**
**F-92500 Rueil Malmaison (FR)**
Inventeur : **Leporq, Serge**
**6, rue Villandry**
**F-78200 Mantes La Ville (FR)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé d'obtention d'au moins une oléfine tertiaire, très pure, par décomposition de l'éther correspondant en présence d'au moins un catalyseur particulier à base de silice et, éventuellement, en absence de vapeur d'eau.

Les oléfines tertiaires obtenues selon la présente invention ont pour formule

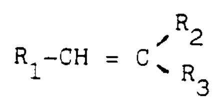

et sont préparées à partir des éthers correspondants de formule

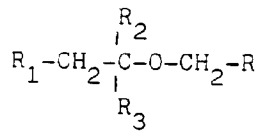

où R et $R_1$ , identiques ou différents, sont chacun choisis dans le groupe formé par l'atome d'hydrogène, les radicaux alkyles, arylalkyles, aryles et alkylaryles et, $R_2$ et $R_3$, identiques ou différents, sont chacun choisis dans le groupe formé par les radicaux alkyles, arylalkyles, aryles et alkylaryles.

Les oléfines tertiaires constituent une matière première très importante pour la préparation de certains produits chimiques, notamment de polymères; il est donc essentiel de disposer d'oléfines tertiaires très pures.

Il est connu que, par réaction d'une oléfine ou d'un mélange d'oléfines avec un alcool primaire en présence d'un acide tel que l'acide sulfurique ou d'un solide ayant une acidité appropriée, on peut obtenir l'éther ou les éthers correspondants. De plus, la vitesse de cette réaction dépendant des conditions opératoires et également de la nature des radicaux R, $R_1$, $R_2$ et $R_3$, il est possible de faire réagir sélectivement, dans une coupe oléfinique telle qu'une coupe issue du vapocraquage, du craquage thermique ou catalytique, les oléfines tertiaires avec au moins un alcool primaire. Le tertio-alkyl-alkyl-éther alors formé peut être aisément séparé des oléfines n'ayant pas réagi, puis décomposé pour redonner l'oléfine tertiaire correspondante purifiée.

Il a déjà été décrit dans l'art antérieur des procédés catalytiques de production d'oléfines tertiaires par décomposition catalytique des éthers correspondants.

Toutefois, cette réaction étant favorisée à haute température, certains catalyseurs de l'art antérieur conduisent à la formation de dialkyléthers et d'eau à partir de l'alcool primaire et donc à une perte plus ou moins importante de l'alcool, grévant ainsi l'économie d'un procédé dans lequel l'alcool est habituellement recyclé à la section de synthèse du tertio-alkyl-alkyl éther.

Une autre réaction parasite favorisée par certains catalyseurs de l'art antérieur est la formation d'oligomères à partir de l'oléfine, qui provoque notamment une chute sensible de la pureté de l'oléfine produite.

La demanderesse a déjà proposé un procédé d'obtention d'une oléfine à partir de l'éther correspondant dans lequel il est remédié en grande partie aux inconvénients précités (US-A-4395580). Ce procédé consiste à opérer à l'aide d'au moins un catalyseur possédant des caractéristiques acides optimisées et en présence de vapeur d'eau. Cette addition de vapeur d'eau permet certes une augmentation des rendements en alcool et oléfine, mais complique le schéma du procédé et engendre un surcoût non négligeable.

Il a été également revendiqué la décomposition de tertioalkyléthers sur un catalyseur à base d'alumine dopée par de la silice (US-A-4006198); toutefois, ce catalyseur conduit à la formation de dialkyléthers dès que la température de réaction est élevée.

Il a aussi été proposé la préparation d'oléfines tertiaires par décomposition des éthers correspondants en présence de catalyseurs à base de silice dopée par de l'alumine ou un oxyde d'un élément choisi parmi le chrome, le béryllium, le titane, le vanadium, le manganèse, le fer, le cobalt, le zinc, le zirconium, le rhodium, l'argent, l'étain, l'antimoine, le bore (US-A-4254296). Mais, comme l'indiquent les auteurs de US-A-4254296 dans la demande de brevet WO 87/00166, ces catalyseurs sont difficiles à préparer et présentent un coût de production très élevé. De plus, ils possèdent une durée de vie relativement faible, nécessitant une augmentation de la température de réaction et entraînant la formation de produits indésirables.

La demande de brevet WO 87/00166 décrit des catalyseurs améliorés pour cette application, constitués de silice modifiée par ajout de 0,1 à 1,5% en poids d'alumine par rapport à la silice. Ces catalyseurs, bien que plus stables que les précédents, présentent toutefois encore une dégradation des performances. Le maintien de la conversion au-dessus de 70% nécessite en effet l'augmentation de la température dans la gamme 130 à 350°C et, dès 3000 heures de fonctionnement, il convient d'opérer à des températures de 250°C pour maintenir cette conversion, ce qui engendre une chute du rendement en méthanol en deçà de 99%.

La présente invention permet de remédier en grande partie à tous les inconvénients précédemment cités.

Elle consiste à opérer en présence d'au moins un catalyseur particulier et, de préférence, en l'absence de vapeur d'eau, ledit catalyseur étant constitué de silice modifiée par l'addition d'au moins un élément ou composé d'un élément choisi dans le groupe constitué par le lithium, le rubidium, le césium, le magnésium, le calcium, le strontium, le baryum, le gallium, les lanthanides (La, Ce, Pr, Nd, Pm, Sm, Eu, Gd, Tb, Dy, Ho, Er, Tm, Yb, Lu) et les uranides (U, Np, Pu, Am), de préférence dans le groupe constitué par le rubidium, le césium, le magnésium, le calcium, le strontium, le baryum, le gallium, le lanthane, le cérium, le praséodyme, le néodyme et l'uranium, de manière encore plus préférée dans le groupe constitué par le rubidium, le magnésium, le calcium, le lanthane et le cérium, et, éventuellement, par l'addition (supplémentaire) d'au moins un élément ou composé d'un élément choisi dans le groupe constitué par l'aluminium, le titane et le zirconium.

La silice utilisée possède de préférence, après incorporation du ou des éléments ou composés d'éléments cités précédemment et après traitement thermique approprié, une surface spécifique d'au moins 100 $m^2/g$ et, plus particulièrement, comprise entre 150 et 700 $m^2/g$.

Le ou les éléments (ou leurs composés) ajoutés à la silice, qu'on appelle ici encore éléments ou agents additonnels ou encore éléments ou agents modificateurs de la silice de base, peuvent être introduits en utilisant toute méthode connue de l'homme de l'art telle que, par exemple, l'échange ionique, l'imprégnation à sec, le mélange mécanique ou le greffage de complexes organométalliques; mais on préfère généralement les introduire en imprégnant ladite silice de base avec au moins une solution contenant le ou les (composés d') éléments que l'on veut ajouter sous forme de leurs sels ou d'autres dérivés organiques ou inorganiques solubles dans le solvant d'imprégnation choisi.

Ainsi, une méthode de préparation du catalyseur employé dans l'invention consiste par exemple à imprégner le support silice au moyen d'au moins une solution aqueuse (ou dans au moins un solvant approprié) contenant le ou les agents modificateurs que l'on veut introduire, ce ou ces agents modificateurs étant utilisés sous forme, par exemple, d'un halogénure, d'un nitrate, d'un acétate, d'un oxalate, d'un sulfate, d'un complexe contenant ledit ou lesdits agents, par exemple, un complexe formé avec l'acide oxalique et les oxalates, avec l'acide citrique et les citrates, l'acide tartrique et les tartrates, avec d'autres polyacides et acides alcools et leurs sels, les acétyl acétonates, et tout autre dérivé inorganique ou organométallique contenant le(s)dit(s) agent(s) modificateur(s) choisi(s).

L'élément ou les éléments modificateurs choisis étant déposés sur la silice, le produit obtenu est ensuite traité thermiquement, c'est-à-dire séché, par tout moyen connu de l'homme de l'art, par exemple sous un courant d'azote ou d'air, puis calciné par exemple sous un courant d'air ou d'azote à une température comprise par exemple entre 300 et 800°C.

La teneur, exprimée en poids d'oxyde par rapport au poids de silice, du ou des éléments ou composés d'éléments modificateurs déposés sur la surface de la silice (et donc contenus dans le catalyseur) est comprise habituellement entre 0,01 et 35%, de préférence entre 0,05 et 25%.

La réaction de décomposition du ou des éthers selon la présente invention est effectuée à une température généralement comprise entre 100 et 500°C et, préférentiellement, entre 130 et 350°C. Si la décomposition des éthers se déroule déjà avec un bon rendement à la pression atmosphérique, on préfère généralement opérer à une pression au moins égale à celle de la tension de vapeur de l'oléfine que l'on désire obtenir, à la température de condensation prévue. On opère habituellement sous une pression comprise entre 0,11 et 2 MPa et, de préférence, entre 0,4 et 1,5 MPa.

Le débit d'éther (s), exprimé en volume de charge liquide par volume de catalyseur et par heure (vitesse spatiale liquide horaire ou VVH) est généralement comprise entre 0,1 et 200 et, de préférence, entre 0,7 et 30.

Le procédé selon l'invention est particulièrement adapté pour l'obtention à l'état pur d'oléfines tertiaires de formule

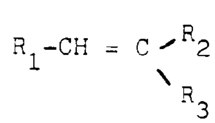

à partir des éthers correspondants de formule

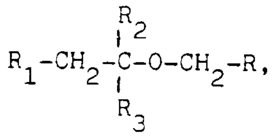

où R et $R_1$, identiques où différents, sont chacun choisis dans le groupe formé par l'atome d'hydrogène, les radicaux méthyle, éthyle, n-propyle et isopropyle et, $R_2$ et $R_3$, identiques ou différents, sont chacun choisis dans le groupe formé par les radicaux méthyle, éthyle, n-propyle et isopropyle.

L'alcool primaire récupéré après décomposition de l'éther contient de préférence 1 à 6 atomes de carbone.

Le procédé selon l'invention peut notamment s'appliquer à la décomposition du méthyl tertiobutyl éther (MTBE) ou de l'éthyl tertiobutyl éther (ETBE) en vue de l'obtention d'isobutène pure (et de méthanol ou d'éthanol).

La réaction de décomposition des éthers en alcools primaires et en oléfines tertiaires selon le procédé de la présente invention est pratiquement quantitative et le taux de récupération de l'alcool et de l'oléfine sont très proches de 100%.

Les exemples suivants illustrent l'invention sans toutefois en limiter la portée.

EXEMPLE 1 : Préparation d'un catalyseur A conforme à celui employé dans l'invention.

50 g de silice de surface spécifique 250 $m^2/g$ et de volume poreux 1,32 $cm^3/g$ sont mis en contact dans un malaxeur rotatif avec une solution aqueuse $S_1$ de 65 $cm^3$ contenant 25 g de nitrate de magnésium hexahydraté, puis la solution est lentement évaporée à sec à 80°C.

La silice imprégnée ainsi obtenue est ensuite séchée pendant environ 1 heure à 100°C, puis pendant environ 16 heures à 150°C, et enfin calcinée pendant environ 3 heures à 600°C.

Le catalyseur A obtenu présente une surface spécifique égale à 220 $m^2/g$ et contient 7,7 % en poids de MgO (par rapport au poids de silice).

EXEMPLE 2 : Préparation d'un catalyseur B conforme à celui employé dans l'invention.

La préparation du catalyseur B diffère de celle décrite dans l'exemple 1 en ce qu'on utilise, à la place de la solution aqueuse $S_1$, une solution aqueuse $S_2$ de 65 $cm^3$ contenant 1,6 g de nitrate de magnésium hexahydraté.

Le catalyseur B obtenu présente une surface spécifique égale à 245 $m^2/g$ et contient 0,46% en poids de MgO (par rapport au poids de silice).

EXEMPLE 3 : Préparation d'un catalyseur C conforme à celui employé dans l'invention.

On prépare une solution aqueuse $S_3$ de 195 $cm^3$ contenant 67 g de nitrate de magnésium hexahydraté. Cette solution est séparée en trois solutions $Z_1$, $Z_2$ et $Z_3$ de 65 $cm^3$ chacune.

50 g de silice de surface spécifique 250 $m^2/g$ et de volume poreux 1,32 $cm^3/g$ sont mis en contact dans un malaxeur rotatif avec la solution $Z_1$ (étape a)) , puis la solution est lentement évaporée à sec à 80°C (étape b)).

La silice imprégnée ainsi obtenue est ensuite séchée pendant environ 1 heure à 100°C (étape c)), puis pendant environ 16 heures à 150°C (étape d)), et enfin calcinée pendant environ 3 heures à 600°C (étape e)).

La silice modifiée ainsi obtenue est alors mise en contact dans un malaxeur rotatif avec la solution $Z_2$ (étape a)), puis subit à nouveau les étapes b) à e). Après cette deuxième imprégnation, une troisième imprégnation est enfin effectuée avec la solution $Z_3$ selon les étapes a) à e).

Le catalyseur C ainsi obtenu présente une surface spécifique égale à 210 $m^2/g$ et contient 20,0% en poids de MgO (par rapport au poids de silice).

EXEMPLE 4 : Préparation d'un catalyseur D conforme à celui employé dans l'invention.

Le catalyseur A préparé dans l'exemple 1 est traité par une solution aqueuse $S_4$ de 60 $cm^3$ contenant 1,4 g de nitrate d'aluminium nonahydraté, puis la solution est lentement évaporée à sec à 80°C.

Le solide ainsi obtenu est ensuite séché pendant environ 1 heure à 100°C, puis pendant environ 16 heures à 150°C, et enfin calciné pendant environ 3 heures à 600°C.

Le catalyseur D ainsi obtenu présente une surface spécifique égale à 240 $m^2/g$ et contient 7,7% en poids

de MgO et 0,37% en poids de $Al_2O_3$ (par rapport au poids de silice).

EXEMPLE 5 : Préparation d'un catalyseur E conforme à celui employé dans l'invention.

La préparation du catalyseur E diffère de celle décrite dans l'exemple 4 en ce qu'on utilise, à la place de la solution aqueuse $S_4$, une solution aqueuse $S_5$ de 60 $cm^3$ contenant 0,30 g de nitrate de zirconyle dihydraté.

Le catalyseur E obtenu présente une surface spécifique égale à 235 $m^2/g$ et contient 7,7 % en poids de MgO et 0,27 % en poids de $ZrO_2$ (par rapport au poids de silice).

EXEMPLE 6 : Préparation d'un catalyseur F conforme à celui employé dans l'invention.

La préparation du catalyseur F diffère de celle décrite dans l'exemple 1 en ce qu'on utilise, à la place de la solution aqueuse $S_1$, une solution aqueuse $S_6$ de 65 $cm^3$ contenant 6,8 g de nitrate de lanthane hexahydraté.

Le catalyseur F obtenu présente une surface spécifique égale à 210 $m^2/g$ et contient 4,6 % en poids de $La_2O_3$ (par rapport au poids de silice).

EXEMPLE 7 : Préparation d'un catalyseur G conforme à celui employé dans l'invention.

La préparation du catalyseur G diffère de celle décrite dans l'exemple 1 en ce qu'on utilise, à la place de la solution aqueuse $S_1$, une solution aqueuse $S_7$ de 65 $cm^3$ contenant 1,6 g de nitrate de rubidium.

Le catalyseur G obtenu présente une surface spécifique égale à 247 $m^2/g$ et contient 2,0 % en poids de $Rb_2O$ (par rapport au poids de silice).

EXEMPLE 8 : Catalyseur H non conforme à celui employé dans l'invention.

Le catalyseur H est constitué de silice non modifiée, de surface spécifique 250 $m^2/g$ et de volume poreux 1,32 $cm^3/g$ (il correspond à la matière première utilisée dans les exemples 1 à 7).

EXEMPLE 9 : Préparation d'un catalyseur I non conforme à celui employé dans l'invention.

La préparation du catalyseur I diffère de celle décrite dans l'exemple 1 en ce qu'on utilise, à la place de la solution aqueuse $S_1$, une solution aqueuse $S_9$ identique à la solution aqueuse $S_4$ décrite dans l'exemple 4.

Le catalyseur I obtenu présente une surface spécifique égale à 250 $m^2/g$ et contient 0,37 % en poids de $Al_2O_3$ (par rapport au poids de silice).

EXEMPLE 10 : Préparation d'un catalyseur J non conforme à celui employé dans l'invention.

La préparation du catalyseur J est effectuée selon le protocole opératoire suivant (qui correspond à celui décrit dans le brevet US-A-4254296) :

40 g d'orthosilicate d'éthyle sont chauffés à 80°C sous azote, puis mis en contact avec 100 ml d'une solution aqueuse à 20% en poids d'hydroxyde de tétrapropyl ammonium sous atmosphère d'azote et sous agitation, la température étant maintenue à 80°C.

Lorsque le mélange devient homogène et limpide, on ajoute 4 g de nitrate de béryllium tétrahydraté dissous dans 80 ml d'éthanol et 1,5 g de soude dissoute dans 10 ml d'eau distillée.

Un gel compacte se forme, auquel on ajoute de l'eau distillée afin d'amener le volume global à 200 ml.

L'ensemble est ensuite porté à ébullition sous forte agitation afin de terminer l'hydrolyse et d'évacuer l'éthanol. Le gel est alors converti en une poudre blanche. De l'eau distillée est à nouveau ajoutée afin de compléter le volume à 150 ml, et l'ensemble est porté à 155°C pendant 17 jours dans un autoclave.

Le solide formé est ensuite séparé par centrifugation, le gâteau est lavé à l'eau distillée et centrifugé quatre fois, puis séché à 120°C dans une étuve, et enfin calciné 16 heures à 550°C sous balayage d'air.

Le solide obtenu est ensuite à nouveau lavé trois fois par remise en suspension dans une solution aqueuse d'acétate d'ammonium portée à ébullition. Le solide est enfin calciné à 550°C pendant 6 heures.

Le catalyseur J ainsi obtenu présente une surface spécifique égale à 470 $m^2/g$ et contient 3,2% en poids de BeO (par rapport au poids de silice).

EXEMPLE 11 : Préparation d'un catalyseur K non conforme à celui employé dans l'inventaire.

50 g de silice de surface spécifique 60 $m^2/g$ et de volume poreux 0,50 $cm^3/g$ sont mis en contact dans un

malaxeur rotatif avec une solution aqueuse $S_{11}$ de 25 $cm^3$ contenant 15 g de nitrate de magnésium hexahydraté, puis la solution est lentement évaporée à sec à 80°C.

La silice imprégnée ainsi obtenue est ensuite séchée pendant environ 1 heure, puis pendant environ 16 heures à 150°C, et enfin calcinée pendant environ 3 heures à 600°C.

Le catalyseur K ainsi obtenu présente une surface spécifique égale à 50 $m^2/g$ et contient 4,6 % en poids de MgO (par rapport au poids de silice).

EXEMPLE 12 : Préparation d'un catalyseur L non conforme à celui employé dans l'invention.

La préparation du catalyseur L diffère de celle décrite dans l'exemple 3 en ce qu'on utilise, à la place de la solution aqueuse $S_3$, une solution aqueuse $S_{12}$ de 195 $cm^3$ contenant 130 g de nitrate de magnésium hexahydraté.

Le catalyseur L obtenu présente une surface spécifique égale à 200 $m^2/g$ et contient 40,0% en poids de MgO (par rapport au poids de silice).

EXEMPLE 13 (selon l'invention)

Les catalyseurs A à G sont chargés dans un réacteur, traités pendant 2 heures sous azote sec, puis testés dans la réaction de décomposition du méthyl tertiobutyl éther (MTBE) en méthanol et isobutène dans les conditions opératoires suivantes :

Température : 170 à 250°C
Pression : 0,7 MPa
VVH : 1 ou $2h^{-1}$

Le tableau 1 résume les résultats obtenus avec ces différents catalyseurs aux conditions de température (T) et de VVH indiquées.

Les concentrations en diméthyl-éther (DME) produit y sont également mentionnées (ppm).

**TABLEAU 1**

| CATALYSEUR | T (°C) | VVH ($h^{-1}$) | DUREE DU TEST (h) | CONVERSION DU MTBE (%) | METHANOL RECUPERE (% pds) | ISOBUTENE RECUPERE (% pds) | DME PRODUIT (ppm) |
|---|---|---|---|---|---|---|---|
| A | 170 | 1 | 200 | 79 | 99,9 | 99,7 | 60 |
| A | 170 | 2 | 200 | 75,0 | 100,0 | 99,9 | 45 |
| A | 220 | 2 | 1000 | 74,5 | 99,9 | 99,9 | 376 |
| A | 250 | 2 | 5000 | 78,2 | 99,3 | 99,9 | 1450 |
| B | 170 | 2 | 200 | 69,5 | 98,9 | 99,5 | 2050 |
| C | 170 | 2 | 200 | 73,0 | 100,0 | 99,9 | 35 |
| D | 170 | 2 | 200 | 82,0 | 99,8 | 99,8 | 147 |
| E | 170 | 2 | 200 | 78,0 | 99,9 | 99,8 | 68 |
| F | 170 | 2 | 100 | 72,8 | 99,9 | 99,9 | 56 |
| G | 170 | 2 | 100 | 73,6 | 99,8 | 99,7 | 173 |

EXEMPLE 14 (comparatif)

Les catalyseurs H à L sont chargés dans un réacteur, traités pendant 2 heures sous azote sec, puis testés dans la réaction de décomposition du méthyl tertiobutyl éther (MTBE) en méthanol et en isobutène à la pression de 0,7 MPa.

Le tableau 2 précise les conditions opératoires utilisées ainsi que les résultats obtenus.

La comparaison entre les tableaux 1 et 2 montre que l'on obtient de meilleurs résultats avec les catalyseurs conformes à ceux préconisés par l'invention.

**TABLEAU 2**

| CATALYSEUR | T (C°) | VVH ($h^{-1}$) | DUREE DU TEST (h) | CONVERSION DU MTBE (%) | METHANOL RECUPERE (% pds) | ISOBUTENE RECUPERE (% pds) | DME PRODUIT (ppm) |
|---|---|---|---|---|---|---|---|
| H | 170 | 2 | 200 | 51,8 | 98,6 | 99,5 | 2550 |
| I | 170 | 2 | 200 | 63,0 | 99,8 | 99,5 | 150 |
| I | 220 | 2 | 1000 | 51,1 | 99,2 | 99,7 | 1500 |
| J | 170 | 2 | 200 | 64,6 | 99,7 | 99,4 | 650 |
| J | 220 | 2 | 1000 | 63,7 | 99,5 | 99,2 | 1000 |
| K | 170 | 2 | 200 | 41,0 | 99,8 | 99,5 | 2130 |
| L | 170 | 2 | 200 | 56,0 | 99,9 | 99,9 | 50 |

## Revendications

1. Procédé d'obtention d'au moins une oléfine tertiaire de formule

$$R_1-CH = C \begin{matrix} R_2 \\ R_3 \end{matrix}$$

par décomposition de l'éther correspondant de formule

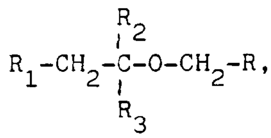

où R et $R_1$, identiques ou différents, sont chacun choisis dans le groupe formé par l'atome d'hydrogène, les radicaux alkyles, arylalkyles, aryles et alkylaryles et, $R_2$ et $R_3$, identiques ou différents, sont chacun choisis dans le groupe formé par les radicaux alkyles, arylalkyles, aryles et alkylaryles, en présence d'au moins un catalyseur constitué de silice modifiée par l'addition d'au moins un élément ou composé d'un élément choisi dans le groupe constitué par le lithium, le rubidium, le césium, le magnésium, le calcium, le strontium, le baryum, le gallium, les lanthanides et les uranides.

2. Procédé selon la revendication 1 dans lequel on opère en absence de vapeur d'eau.

3. Procédé selon l'une des revendications 1 et 2 dans lequel ledit catalyseur est constitué de silice modifiée par l'addition d'au moins un élément ou composé d'un élément choisi dans le groupe constitué par le rubidium, le césium, le magnésium, le calcium, le strontium, le baryum, le gallium, le lanthane, le cérium, le praséodyme, le néodyme et l'uranium.

4. Procédé selon l'une des revendications 1 et 2 dans lequel ledit catalyseur est constitué de silice modifiée par l'addition d'au moins un élément ou composé d'un élément choisi dans le groupe constitué par le rubidium, le magnésium, le calcium, le lanthane et le cérium.

5. Procédé selon l'une des revendications 1 et 4 dans lequel la silice est modifiée en outre par l'addition d'au moins un élément ou composé d'un élément choisi dans le groupe constitué par l'aluminium, le titane et le zirconium.

6. Procédé selon l'une des revendications 1 à 5 dans lequel la teneur, exprimée en poids d'oxyde par rapport au poids de silice, dudit ou desdits éléments ou composés d'éléments contenus dans ledit catalyseur est comprise entre 0,01 et 35%.

7. Procédé selon la revendication 6 dans lequel ladite teneur est comprise entre 0,05 et 25%.

8. Procédé selon l'une des revendications 1 à 7 dans lequel ladite silice, après incorporation dudit ou desdits éléments ou composés d'éléments, possède une surface spécifique d'au moins 100 $m^2/g$.

9. Procédé selon l'une des revendications 1 à 7 dans lequel ladite silice, après incorporation dudit ou desdits éléments ou composés d'éléments, possède une surface spécifique comprise entre 150 et 700 $m^2/g$.

10. Procédé selon l'une des revendications 1 à 9 dans lequel on opère sous une pression comprise entre 0,11 et 2 MPa, à une température comprise entre 100 et 500°C, avec une vitesse spatiale liquide horaire comprise entre 0,1 et 200 volumes de charge liquide par volume de catalyseur et par heure.

11. Procédé selon l'une des revendications 1 à 9 dans lequel on opère sous une pression comprise entre 0,4 et 1,5 MPa, à une température comprise entre 130 et 350°C, avec une vitesse spatiale liquide horaire comprise entre 0,7 et 30 volumes de charge liquide par volume de catalyseur et par heure.

12. Procédé selon l'une des revendications 1 à 11 dans lequel l'éther de départ est le produit résultant du traitement d'une coupe oléfinique, contenant l'oléfine tertiaire désirée, par un alcool pour obtenir l'éther correspondant à l'oléfine tertiaire désirée, suivi de l'isolement, par distillation, dudit éther.

13. Procédé selon l'une des revendications 1 à 11 dans lequel R et $R_1$, identiques ou différents, sont chacun choisis dans le groupe formé par l'atome d'hydrogène, les radicaux méthyle, éthyle, n-propyle et isopropyle et, $R_2$ et $R_3$, identiques ou différents, sont chacun choisis dans le groupe formé par les radicaux méthyle, éthyle, n-propyle et isopropyle.

14. Procédé selon l'une des revendications 1 à 13 dans lequel ledit éther est le méthyl tertiobutyl éther et ladite oléfine tertiaire est l'isobutène.

**15.** Procédé selon l'une des revendications 1 à 13 dans lequel ledit éther est l'éthyl tertiobutyl éther et ladite oléfine tertiaire est l'isobutène.

## Patentansprüche

**1.** Verfahren zur Herstellung von mindestens einem tertiären Olefin mit der Formel

$$
\begin{array}{l} \qquad\qquad\quad R_2 \\ \qquad\qquad\;\; / \\ R_1-CH = C \\ \qquad\qquad\;\; \backslash \\ \qquad\qquad\quad R_3 \end{array}
$$

durch Zersetzung des entsprechenden Ethers mit der Formel

$$
\begin{array}{c} R_2 \\ | \\ R_1-CH_2-C-O-CH_2-R \\ | \\ R_3 \end{array}
$$

in denen R und $R_1$, identisch oder voneinander verschieden, jeweils aus der vom Wasserstoffatom, den Alkyl-, Arylalkyl-, Aryl- und Alkylarylradikalen gebildeten Gruppe ausgewählt werden und $R_2$ und $R_3$, identisch oder voneinander verschieden, jeder aus der von den Alkyl-, Arylalkyl-, Aryl- und Alkylarylradikalen gebildeten Gruppe ausgewählt werden, in Gegenwart von mindestens einem Katalysator, der aus Siliciumoxid besteht, das durch die Zugabe von mindestens einem Element oder der Verbindung eines Elementes, das aus der von Lithium, Rubidium, Cäsium, Magnesium, Calcium, Strontium, Barium, Gallium, den Lanthaniden und den Actiniden gebildeten Gruppe gewählt wird, modifiziert ist.

**2.** Verfahren nach Anspruch 1, bei dem in Abwesenheit von Wasserdampf gearbeitet wird.

**3.** Verfahren nach einem der Ansprüche 1 und 2, bei dem der besagte Katalysator aus Siliciumoxid besteht, das durch die Zugabe von mindestens einem Element oder der Verbindung eines Elementes, das aus der von Rubidium, Cäsium, Magnesium, Calcium, Strontium, Barium, Gallium, Lanthan, Cer, Praseodym, Neodym und Uran gebildeten Gruppe gewählt wird, modifiziert ist.

**4.** Verfahren nach einem der Ansprüche 1 und 2, bei dem der besagte Katalysator aus Siliciumoxid besteht, das durch die Zugabe von mindestens einem Element oder der Verbindung eines Elementes, das aus der von Rubidium, Magnesium, Calcium, Lanthan und Cer gebildeten Gruppe gewählt wird, modifiziert ist.

**5.** Verfahren nach einem der Ansprüche 1 und 4, bei dem der besagte Katalysator aus Siliciumoxid besteht, das durch die Zugabe von mindestens einem Element oder der Verbindung eines Elementes, das aus der von Aluminium, Titan und Zirkonium gebildeten Gruppe gewählt wird, modifiziert ist.

**6.** Verfahren nach einem der Ansprüche 1 bis 5, bei dem der Gehalt des oder der Elemente oder Verbindungen von Elementen, die in dem besagten Katalysator enthalten sind, ausgedrückt in Gewicht des Oxides bezogen auf das Gewicht des Silciumoxides zwischen einschließlich 0,01 und 35 % liegt.

**7.** Verfahren nach Anspruch 6, bei dem der besagte Gehalt zwischen einschließlich 0,05 und 25 % liegt.

**8.** Verfahren nach einem der Ansprüche 1 bis 7, bei dem das besagte Siliciumoxid nach Aufnahme des oder der Elemente oder Verbindungen von Elementen eine spezifische Oberfläche von mindestens 100 $m^2/g$ besitzt.

**9.** Verfahren nach einem der Ansprüche 1 bis 7, bei dem das besagte Siliciumoxid nach Aufnahme des oder der Elemente oder Verbindungen von Elementen eine spezifische Oberfläche, die zwischen einschließlich 150 und 700 $m^2/g$ liegt, besitzt.

10. Verfahren nach einem der Ansprüche 1 bis 9, bei dem unter einem Druck zwischen einschließlich 0,11 und 2 MPa, einer Temperatur zwischen einschließlich 100 und 500 °C, mit einer stündlichen Flüssigkeitsraumgeschwindigkeit zwischen einschließlich 0,1 und 200 Volumina des flüssigen Einsatzstoffes pro Katalysatorvolumen und Stunde gearbeitet wird.

11. Verfahren nach einem der Ansprüche 1 bis 9, bei dem unter einem Druck zwischen einschließlich 0,4 und 1,5 MPa, einer Temperatur zwischen einschließlich 130 und 350 °C, mit einer stündlichen Füssigkeitsraumgeschwindigkeit zwischen einschließlich 0,7 und 30 Volumina des flüssigen Einsatzstoffes pro Katalysatorvolumen und Stunde gearbeitet wird.

12. Verfahren nach einem der Ansprüche 1 bis 11, bei dem der Ether-Ausgangsstoff das Produkt ist, das aus der Behandlung eines das gewünschte tertiäre Olefin umfassende Olefinschnittes mit einem Alkohol zur Herstellung des dem gewünschten tertiären Olefin entsprechenden Ethers resultiert, gefolgt von der Isolierung des besagten Ethers durch Destillation.

13. Verfahren nach einem der Ansprüche 1 bis 11, bei dem R und $R_1$, identisch oder voneinander verschieden, jeder aus der vom Wasserstoffatom, den Methyl-, Ethyl-, n-Propyl- und iso-Propyl-Radikalen gebildeten Gruppe ausgewählt werden und $R_2$ und $R_3$, identisch oder voneinander verschieden, jeder aus der von den Methyl-, Ethyl-, n-Propyl- und iso-Propyl-Radikalen gebildeten Gruppe ausgewählt werden.

14. Verfahren nach einem der Ansprüche 1 bis 13, bei dem der besagte Ether Methyl-tert.-Butylether und das besagte tertiäre Olefin Isobuten ist.

15. Verfahren nach einem der Ansprüche 1 bis 13, bei dem der besagte Ether Ethyl-tert.-Butylether und das besagte tertiäre Olefin Isobuten ist.

## Claims

1. Process for obtaining at least one tertiary olefin of formula

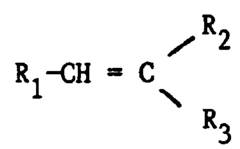

by decomposition of the corresponding ether of formula

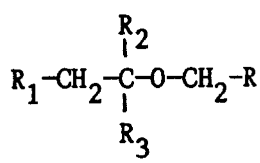

in which R and $R_1$, which can be the same or different, are each chosen from within the group formed by the hydrogen atom, the alkyl, aryl alkyl, aryl and alkyl aryl radicals and $R_2$ and $R_3$, which can be the same or different, are each chosen from within the group formed by the alkyl, aryl alkyl, aryl and alkyl aryl radicals, in the presence of at least one catalyst constituted by silica modified by the addition of at least one element or compound of an element chosen from within the group constituted by lithium, rubidium, cesium, magnesium, calcium, strontium, barium, gallium, lanthanides and uranides.

2. Process according to claim 1, wherein working takes place in the absence of water vapour.

3. Process according to either of the claims 1 and 2, wherein the said catalyst is constituted by silica modified by the addition of at least one element or compound of an element chosen from within the group constituted by rubidium, cesium, magnesium, calcium, strontium, barium, gallium, lanthanum, cerium, praseodymium, neodymium and uranium.

4. Process according to either of the claims 1 and 2, wherein said catalyst is constituted by silica modified by the addition of at least one element or compound of an element chosen from within the group constituted by rubidium, magnesium, calcium, lanthanum and cerium.

5. Process according to either of the claims 1 and 4, wherein the silica is also modified by the addition of at least one element or compound of an element chosen from within the group constituted by aluminium, titanium and zirconium.

6. Process according to any one of the claims 1 to 5, wherein the content, expressed by weight of oxide based on the silica weight, of said element or elements or said compounds of elements contained in said catalyst is between 0.01 and 35%.

7. Process according to claim 6, wherein said content is between 0.05 and 25%.

8. Process according to any one of the claims 1 to 7, wherein said silica, following the incorporation of said element or elements or said compounds of elements, has a specific surface of at least 100 $m^2/g$.

9. Process according to any one of the claims 1 to 7, wherein said silica, following the incorporation of said element or elements, or said compounds of elements, has a specific surface between 150 and 700 $m^2/g$.

10. Process according to any one of the claims 1 to 9, wherein working takes place under a pressure between 0.11 and 2 MPa, at a temperature between 100 and 500°C and with an hourly liquid space velocity between 0.1 and 200 liquid charge volumes per catalyst volume and per hour.

11. Process according to any one of the claims 1 to 9, wherein working takes place under a pressure between 0.4 and 1.5 MPa, a temperature between 130 and 350°C and an hourly liquid space velocity between 0.7 and 30 liquid charge volumes per catalyst volume and per hour.

12. Process according to any one of the claims 1 to 11, wherein the starting ether is the product resulting from the treatment of an olefin fraction, containing the desired tertiary olefin, by an alcohol in order to obtain the ether corresponding to the desired tertiary olefin, followed by distillative separation of the said ether.

13. Process according to any one of the claims 1 to 11, wherein R and $R_1$, which can be the same or different, are each chosen from within the group formed by the hydrogen atom, the methyl, ethyl, n-propyl and isopropyl radicals and $R_2$ and $R_3$, which can be the same or different, are each chosen from within the group formed by the methyl, ethyl, n-propyl and isopropyl radicals.

14. Process according to any one of the claims 1 to 13, wherein the said ether is methyl tert. butyl ether and said tertiary olefin is isobutylene.

15. Process according to any one of the claims 1 to 13, wherein said ether is ethyl tert. butyl ether and said tertiary olefin is isobutylene.